(19) Europäisches Patentamt / European Patent Office / Office européen des brevets

(11) **EP 3 163 163 B1**

(12) **FASCICULE DE BREVET EUROPEEN**

(45) Date de publication et mention
de la délivrance du brevet:
**24.02.2021 Bulletin 2021/08**

(51) Int Cl.:
*F23G 7/00* $^{(2006.01)}$     *G01N 1/32* $^{(2006.01)}$
*G01N 21/85* $^{(2006.01)}$     *G06T 7/00* $^{(2017.01)}$

(21) Numéro de dépôt: **16195584.4**

(22) Date de dépôt: **25.10.2016**

(54) **PROCEDE ET INSTALLATION D'ANALYSE DE METAUX**

VERFAHREN UND ANLAGE ZUR ANALYSE VON METALLEN

METHOD AND FACILITY FOR ANALYSING METALS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.11.2015 FR 1560473**

(43) Date de publication de la demande:
**03.05.2017 Bulletin 2017/18**

(73) Titulaire: **SUEZ Groupe**
**92040 Paris la Défense Cedex (FR)**

(72) Inventeurs:
- **ALLEGRINI, Elisa**
  **75013 Paris (FR)**
- **RENVOISE, Laure**
  **78500 Sartrouville (FR)**
- **PEREGRINA, Carlos**
  **34980 Saint Clement de Riviere (FR)**

(74) Mandataire: **IPAZ**
**16, rue Gaillon**
**75002 Paris (FR)**

(56) Documents cités:
**JP-A- H01 207 649     JP-A- 2009 198 414**

- **SABIR SYED: "Optical and Chemical Methods of Metal Ash Analysis and Tin Recovery", ENVIRONMENTAL MONITORING AND ASSESSMENT ; AN INTERNATIONAL JOURNAL DEVOTED TO PROGRESS IN THE USE OF MONITORING DATA IN ASSESSINGENVIRONMENTAL RISKS TO MAN AND THE ENVIRONMENT, KLUWER ACADEMIC PUBLISHERS, DO, vol. 130, no. 1-3, 21 octobre 2006 (2006-10-21), pages 311-322, XP019500288, ISSN: 1573-2959**
- **NIJHOF G H ET AL: "Magnus separation of dross and bottom ashes and image analysis during separation of non-ferrous metal scrap", LIGHT METALS: PROCEEDINGS OF SESSIONS, TMS ANNUAL MEETING (WARRENDALE, PENNSYLVANIA), MINERALS, METALS AND MATERIALS SOCIETY ALUMINUM COMMITTEE WARRENDALE, PA, US, 1 janvier 2001 (2001-01-01), pages 1125-1129, XP008181275, ISSN: 1096-9586**

EP 3 163 163 B1

**Description**

**Domaine technique**

La présente invention se rapporte au domaine du tri de métaux et au domaine du traitement de déchets, en particulier de déchets urbains.

Plus spécifiquement, la présente invention concerne un procédé et une installation d'analyse de métaux contenus dans un échantillon de fragments métalliques.

**Etat de la technique antérieure**

Dans le domaine du traitement de déchets, en particulier de déchets urbains, il est courant d'incinérer de tels déchets puis de trier les résidus des déchets incinérés afin d'en récupérer des fragments métalliques valorisables, typiquement afin de réutiliser ces fragments métalliques dans le secteur de la construction.

On connaît dans l'état de la technique antérieure des procédés permettant de trier de tels résidus ou plus généralement des fragments métalliques.

Un premier type de procédé connu consiste à analyser des échantillons de fragments métalliques en laboratoire. Une analyse en laboratoire est à la fois coûteuse et peu rapide, nécessitant typiquement plusieurs jours pour fournir un résultat d'analyse. Voir par exemple le document de Sabir Syed « Optical and Chemical Methods of Metal Ash Analysis and Tin Recovery" Environ Monit Assess (2007) 130 :311-322, DOI 10.1007/s10661-00669399-y XP19500288.

Or, dans le domaine industriel, il est important d'obtenir rapidement un résultat d'analyse fiable, au moins pour les raisons suivantes :

- Pour pouvoir régler de manière optimale les paramètres des équipements de tri mécanique ;
- Pour pouvoir contrôler fréquemment les performances de ces équipements, et établir facilement des corrélations entre de telles performances et les déchets ou résidus de déchets qui entrent dans ces équipements de tri ;
- Pour disposer d'une grande quantité de données relatives à la qualité des échantillons de fragments métalliques analysés.

Un deuxième type de procédé connu consiste à réaliser des analyses d'échantillons de fragments métalliques sur site. Les analyses sur site permettent de fournir rapidement un résultat d'analyse mais ne permettent pas de substituer complètement les analyses en laboratoire. En effet, les analyses en laboratoire restent requises pour calibrer les analyses sur site. Toutefois, les analyses sur site permettent d'augmenter la fréquence de contrôle des équipements à moindre coût financier et temporel.

On connaît en particulier, dans l'état de la technique antérieure, des procédés d'analyse sur site, et parfois en ligne, mettant en œuvre des techniques de traitement d'image. Bien que rarement, ces techniques ont déjà été appliquées dans le domaine du traitement de déchets urbains.

Par exemple, une telle application est décrite dans le document Bonifazi & Serranti, Hyperspectral imaging based procédures applied to bottom ash characterization, In Vo-Dinh, Lieberman & Gauglitz (Eds.), Advanced Environmental, Chemical and Biological Sensing Technologies V, Proc. of SPIE Vol. 6755, 67550B, 2007 (ci-après Bonifazi 2007). Bonifazi 2007 décrit une technique d'imagerie hyperspectrale mise en œuvre pour évaluer le contenu de matières organiques résiduelles dans des déchets urbains. Cette technique est toutefois relativement complexe à mettre en œuvre.

Le document ZAR, Methodenband, 2014, http://zar-ch.ch/fileadmin/user_upload/Contentdokumente/Oeffentliche_Dokumente/M ethodenband_DE.pdf (ci-après ZAR 2014), décrit un procédé pour caractériser des métaux contenus dans des résidus issus d'une étape de combustion de déchets urbains. Le procédé de ZAR 2014 met en œuvre, entre autres, une étape d'analyse densimétrique de ces fragments.

Un tel procédé implique des durées d'analyse conséquentes.

Plus généralement, les procédés connus dans l'état de la technique antérieure ne permettent pas de réaliser une analyse rapide et fiable, ou satisfaisante en tant que telle, d'un échantillon de fragments métalliques. Ces procédés ne permettent pas non plus d'estimer sur site les quantités relatives de différents types de métaux contenus dans un tel échantillon de fragments métalliques en répondant aux exigences de rapidité, de fiabilité et de bas coût requises.

Un but de la présente invention est de proposer un procédé d'analyse de métaux capable de surmonter de tels inconvénients.

**Exposé de l'invention**

A cet effet, l'invention propose un procédé d'analyse de métaux comprenant une étape de nettoyage par traitement acide dans laquelle un échantillon de fragments métalliques est immergé, de préférence pendant une durée d'au moins 4 minutes, préférentiellement entre 4 et 6 minutes, plus préférentiellement de 5 minutes, dans une solution acide, comprenant de l'acide nitrique, caractérisé en ce qu'il comprend en outre :

- une étape de numérisation dans laquelle l'échantillon nettoyé de fragments métalliques est numérisé de manière à constituer une image numérique de cet échantillon,
- une étape d'analyse numérique dans laquelle on recherche sur l'image numérique la présence éventuelle d'au moins deux types de métaux dans l'échantillon de fragments métalliques,
- une étape de calcul de proportion dans laquelle on calcule des quantités relatives entre lesdits au moins deux types de métaux ou un rapport de quantités relatives entre lesdits au moins deux types de métaux, lesdits au moins deux métaux comprenant un premier type de métal ledit premier type de métal étant un métal non ferreux léger dont la densité est inférieure à 2800 kg/m$^3$ et un deuxième type de métal ledit deuxième type de métal étant un métal non ferreux lourd dont la densité est supérieure à 2800 kg/m$^3$.

Les étapes d'analyse numérique et/ou de calcul de proportion sont de préférence mises en œuvre uniquement par des moyens techniques comprenant des moyens électroniques et/ou informatiques et/ou logiciels.

Ce procédé permet d'estimer objectivement la proportion relative de différents types de métaux contenus dans l'échantillon de fragments métalliques, en évitant de baser une telle estimation sur une inspection visuelle subjective. Le temps d'analyse est ainsi réduit et la fiabilité de l'analyse améliorée.

De préférence, la recherche sur l'image numérique de présence éventuelle d'au moins deux types de métaux dans l'échantillon de fragments métalliques peut comprendre un ajustement de seuil de couleur et/ou de saturation et/ou de luminosité de l'image numérique.

La solution acide comprend de l'acide nitrique et consiste de préférence en une solution d'acide nitrique.

Un avantage de la solution acide comprenant de l'acide nitrique réside dans une propriété intéressante de l'acide nitrique lui permettant, par l'effet de son action sur l'échantillon de fragments métalliques lors de l'étape de nettoyage, d'augmenter sur l'image numérique de cet échantillon le contraste de couleurs entre métaux non ferreux légers et métaux non ferreux lourds, en particulier entre des alliages d'aluminium et du zinc. Ainsi, le nettoyage par traitement acide permet de fiabiliser l'étape d'analyse numérique et consécutivement le résultat du calcul réalisé dans l'étape de calcul de proportion.

La concentration de la solution acide peut être d'au moins 4 %, de préférence entre 4 % et 6 %. Le ratio entre le volume de la solution acide et la masse de l'échantillon de fragments métalliques peut être d'au moins 0,4 l/kg, de préférence entre 0,4 l/kg et 0,6 l/kg, plus préférentiellement de 0,5 l/kg.

Le ratio entre le volume de la solution acide et la masse de l'échantillon de fragments métalliques peut être d'au moins 0,4 l/kg, de préférence entre 0,4 l/kg et 0,6 l/kg, plus préférentiellement de 0,5 l/kg. Un tel ratio entre 0,4 l/kg et 0,6 l/kg, de préférence sensiblement égal à 0,5 l/kg, permet de recouvrir entièrement l'échantillon d'acide pendant le nettoyage à l'acide.

Dans un mode de mise en œuvre, le premier type de métal est typiquement des alliages d'aluminium, et le deuxième type de métal est typiquement des alliages de cuivre, de plomb, de zinc et de métaux précieux.

De préférence, dans ce mode de mise en œuvre, le premier type de métal peut comprendre de l'aluminium et le deuxième type de métal peut comprendre du zinc et/ou du cuivre. Ces métaux peuvent être distingués pour leur différence de couleurs.

De préférence, l'échantillon de fragments métalliques peut être issu de résidus d'incinération de déchets.

De préférence, le procédé peut comprendre en outre, avant l'étape de nettoyage par traitement acide, une étape de traitement de déchets, l'échantillon de fragments métalliques étant une partie des déchets ainsi traités.

Une telle étape de traitement de déchets peut comprendre :

- une opération de traitement thermique dans laquelle les déchets sont incinérés, les déchets ainsi incinérés générant des résidus solides,
- une opération de tri mécanique dans laquelle les résidus solides sont triés de manière à extraire des composants métalliques à partir des résidus solides, l'échantillon de fragments métalliques étant tous ou une partie de ces composants métalliques ainsi extraits.

Le procédé peut comprendre en outre, avant l'étape de nettoyage par traitement acide, une étape de purification dans laquelle des impuretés sont extraites de l'échantillon de fragments métalliques. Une telle étape de purification améliore l'efficacité du nettoyage par traitement acide. De préférence, l'échantillon de fragments métalliques ayant subi cette étape de purification peut avoir une masse inférieure à 1 kg.

Les impuretés peuvent comprendre des fragments non métalliques.

Dans un mode de réalisation, le procédé peut comprendre en outre, avant l'étape de numérisation et après l'étape de nettoyage par traitement acide, une étape d'étalement dans laquelle l'échantillon de fragments métalliques est étalé sur un support de manière à réduire des chevauchements entre différents fragments de l'échantillon de fragments métalliques.

De préférence, le support peut être choisi pour pouvoir être distingué, sur l'image numérique, desdits au moins deux types de métaux dans l'échantillon de fragments métalliques lors de l'étape d'analyse numérique. Dans un mode de réalisation, le support est de couleur bleu ; avantageusement, le support étant de couleur bleu, cette couleur permet de distinguer le support dudit échantillon. Dans un mode de réalisation, le support est en plastique.

Lors de l'étape de calcul de proportion, le rapport $P_{i,j}$ de quantités relatives entre lesdits au moins deux types de métaux peut être calculé avec la formule suivante :

$$P_{i,j} = \frac{np_i * d_i}{np_i * d_i + np_j * d_j}$$

où : $i$ est un premier type de métal, $j$ est un deuxième type de métal, $np_i$ est un nombre de pixels de l'image numérique correspondant au premier type de métal d'après l'étape d'analyse numérique, $np_j$ est un nombre de pixels de l'image numérique correspondant au deuxième type de métal d'après l'étape d'analyse numérique, $d_i$ correspond à la densité réelle du premier type de métal, $d_j$ correspond à la densité réelle du deuxième type de métal. La densité réelle d'un corps s'exprime par le rapport entre la masse d'un certain volume de ce corps et la masse du même volume d'eau, dans des conditions identiques de température et de pression. Il s'agit d'une propriété propre à chaque matériau.

La présente invention concerne aussi une installation d'analyse de métaux comprenant des moyens de nettoyage par traitement acide agencés pour immerger un échantillon de fragments métalliques dans une solution acide comprenant de l'acide nitrique, de préférence dans un récipient résistant à la solution acide tel qu'un récipient en plastique ou autre, caractérisé en ce qu'elle comprend en outre :

- des moyens de numérisation agencés pour numériser l'échantillon nettoyé de fragments métalliques de manière à constituer une image numérique de cet échantillon,
- des moyens d'analyse numérique agencés pour rechercher sur l'image numérique la présence éventuelle d'au moins deux types de métaux dans l'échantillon de fragments métalliques,
- des moyens de calcul de proportion agencés pour calculer des quantités relatives entre lesdits au moins deux types de métaux ou un rapport de quantités relatives entre lesdits au moins deux types de métaux, lesdits au moins deux types de métaux comprenant un premier type de métal, ledit premier type de métal étant un métal non ferreux léger dont la densité est inférieure à 2800 kg/m$^3$ et un deuxième type de métal, ledit deuxième type de métal étant un métal non ferreux lourd dont la densité est supérieure à 2800 kg/m$^3$.

De préférence, les moyens d'analyse numérique peuvent comprendre des moyens d'ajustement agencés pour permettre un ajustement de seuil de couleur et/ou de saturation et/ou de luminosité de l'image numérique lors de la recherche sur l'image numérique de présence éventuelle d'au moins deux types de métaux dans l'échantillon de fragments métalliques.

De préférence, l'installation peut comprendre en outre des moyens de traitement de déchets en amont des moyens de nettoyage par traitement acide.

De tels moyens de traitement de déchets peuvent comprendre :

- des moyens de traitement thermique agencés pour incinérer les déchets et générer ainsi des résidus solides de ces déchets,
- un centre de tri agencé pour trier les résidus solides et pour extraire des composants métalliques à partir des résidus solides, l'échantillon de fragments métalliques étant tous ou une partie de ces composants métalliques ainsi extraits.

L'installation peut comprendre en outre, en amont des moyens de nettoyage par traitement acide, des moyens de purification agencés pour extraire des impuretés de l'échantillon de fragments métalliques.

Dans un mode de réalisation, l'installation peut comprendre en outre, en aval des moyens de nettoyage par traitement acide, un support agencé pour recevoir l'échantillon de fragments métalliques et des moyens d'étalement agencés pour étaler l'échantillon de fragments métalliques sur le support de manière à réduire des chevauchements entre différents fragments de l'échantillon de fragments métalliques.

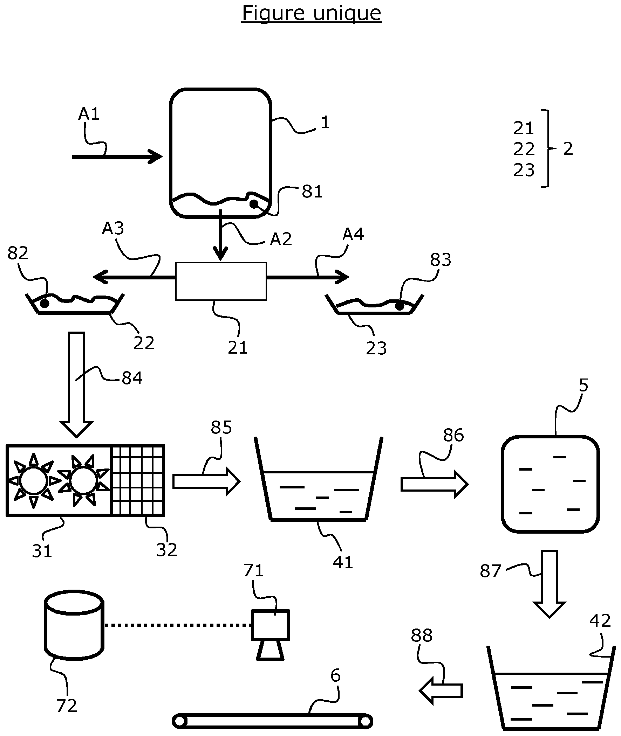

**Description de la figure et de modes de réalisation**

D'autres avantages et particularités de l'invention apparaîtront à la lecture de la description détaillée de mises en œuvre et de modes de réalisation nullement limitatifs, et de la figure unique annexée qui représente de manière schématique une installation d'analyse de métaux pour la mise en œuvre du procédé selon l'invention.

Les modes de réalisation décrits ci-après étant nullement limitatifs, on pourra notamment considérer des variantes de l'invention ne comprenant qu'une sélection de caractéristiques décrites, isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

Un exemple d'installation et de procédé selon l'invention est illustré sur la figure annexée qui schématise des moyens de traitement thermique 1 et un centre de tri 2 permettant de constituer un échantillon de fragments métalliques à partir de déchets, de préférence à partir de déchets urbains.

Constitution d'un échantillon de fragments métalliques

Dans l'exemple non limitatif illustré sur la figure, les déchets sont tout d'abord acheminés, étape A1, dans les moyens de traitement thermique 1 agencés pour réaliser une opération de traitement thermique dans laquelle les déchets sont incinérés. Ces moyens de traitement thermique 1 consistent par exemple en un incinérateur permettant de soumettre les déchets à une réaction de combustion. Les déchets ainsi incinérés génèrent des résidus solides 81 ou mâchefers.

Les résidus solides 81 générés par cette opération de traitement thermique sont ensuite acheminés, étape A2, vers le centre de tri 2 permettant de réaliser une opération de tri mécanique. Dans cet exemple, l'opération de tri mécanique consiste à trier les résidus solides 81 de manière à extraire des composants métalliques non ferreux 82 à partir des résidus solides 81. Pour ce faire, le centre de tri 2 comprend par exemple un équipement de tri 21, par exemple un séparateur à courants de Foucault. Cet équipement de tri 21 permet, d'une part, d'acheminer, étape A3, lesdits composants métalliques non ferreux 82 vers une première section de tri 22 et, d'autre part, d'acheminer, étape A4, la partie restante 83 des résidus solides 81 - c'est-à-dire la partie des résidus solides 81 desquels ont été séparés les composants métalliques non ferreux 82 extraits sous l'action de l'équipement de tri 21 - vers une deuxième section de tri 23.

Suivant une première variante de l'invention, les composants métalliques non ferreux 82 ainsi extraits des résidus solides 81 par cette opération de tri mécanique constituent en tout ou en partie un échantillon de fragments métalliques 84, dont la masse est de préférence comprise entre 1 kg et 5 kg.

Cet échantillon de fragments métalliques 84 pouvant donc comprendre des métaux non ferreux lourds et des métaux non ferreux légers, les étapes ultérieures du procédé de l'invention (décrites dans les sections suivantes) peuvent être mises en œuvre pour quantifier la proportion desdits métaux lourds et desdits métaux légers dans cet échantillon.

Suivant une deuxième variante de l'invention, les composants métalliques non ferreux 82 ainsi extraits des résidus solides 81 peuvent être soumis à une étape de tri supplémentaire (non représentée) dans laquelle sont séparées, par exemple à l'aide d'une table densimétrique :

- d'une part, les composants métalliques non ferreux légers comprenant majoritairement des métaux légers, dont la densité est typiquement inférieure à 2800 kg/m$^3$,
- d'autre part, les composants métalliques non ferreux lourds comprenant majoritairement des métaux lourds, dont la densité est typiquement supérieure à 2800 kg/m$^3$.

Suivant cette deuxième variante, l'échantillon de fragments métalliques 84 peut être constitué :

- de tout ou partie desdits composants métalliques non ferreux légers, ou
- de tout ou partie desdits composants métalliques non ferreux lourds.

En résumé, l'opération de traitement thermique et l'opération de tri mécanique constituent une étape de traitement de déchets, cette étape de traitement de déchets permettant de constituer un échantillon de fragments métalliques 84 à partir de déchets, par exemple de déchets urbains.

Purification de l'échantillon

Dans cet exemple, à l'issue de l'étape de traitement de déchets, l'échantillon de fragments métalliques 84 subit une étape de purification dans laquelle des impuretés sont extraites de cet échantillon de fragments métalliques 84. Les impuretés comprennent typiquement des fragments non métalliques.

L'extraction d'impuretés dans l'échantillon de fragments métalliques 84 est par exemple réalisée à l'aide d'un concasseur 31, par exemple à mâchoires.

Les fragments non métalliques concassés sont par exemple séparés de l'échantillon de fragments métalliques 84 à l'aide d'un tamis 32 ayant par exemple des ouvertures de 1 mm.

Ces opérations de concassage et/ou de tamisage peuvent être répétées si un trop grand nombre d'impuretés ne sont pas éliminées lors d'une opération de tamisage.

L'échantillon de fragments métalliques 85 ayant subi cette étape de purification a de préférence une masse inférieure à 1 kg.

Cet échantillon de fragments métalliques 85 est ensuite rincé à l'eau à l'aide d'un premier moyen de rinçage 41, par exemple par projection d'eau sur l'échantillon disposé sur un tamis (non représenté), afin d'en extraire encore davantage d'impuretés.

Nettoyage par traitement acide de l'échantillon

A l'issue de l'étape de purification, l'échantillon de fragments métalliques 86 est immergé dans une solution acide, de préférence pendant une durée d'au moins 4 minutes, pour subir une étape de nettoyage par traitement acide.

Cette étape de nettoyage est réalisée par des moyens de nettoyage par traitement acide 5 comprenant par exemple un récipient en plastique.

La solution acide comprend de l'acide nitrique. La concentration de la solution acide est de préférence comprise entre 4 % et 6 %, plus préférentiellement de 5 %. De préférence, le ratio entre le volume de solution acide et la masse de l'échantillon de fragments métalliques 86 est de préférence entre 0,4 l/kg et 0,6 l/kg, plus préférentiellement de 0,5 l/kg.

Dans cet exemple, l'échantillon de fragments métalliques 87 ayant subi l'étape de nettoyage par traitement acide est rincé à l'eau à l'aide d'un deuxième moyen de rinçage 42, par exemple par projection d'eau sur l'échantillon disposé sur un tamis (non représenté), afin de faciliter sa manipulation.

Dans le cas où l'échantillon de fragments métalliques 88 sortant du deuxième moyen de rinçage 42 serait insuffisamment nettoyé, l'étape de nettoyage par traitement acide, avec un rinçage subséquent à l'eau, pourrait être répétée.

Analyse de l'échantillon

L'échantillon de fragments métalliques 88 ayant subi l'étape de purification comprend plusieurs types de métaux, un métal non ferreux léger, de densité inférieure à 2800 kg/m$^3$, et un métal non ferreux lourd, de densité supérieure à 2800 kg/m$^3$, par exemple de l'aluminium et du zinc, et/ou de l'aluminium et du cuivre.

Afin de faciliter son analyse, l'échantillon de fragments métalliques 88 est de préférence étalé sur un support 6 de manière à réduire des chevauchements entre différents fragments de l'échantillon 88 (étape d'étalement).

L'échantillon de fragments métalliques 88 subit alors une étape de numérisation dans laquelle cet échantillon est numérisé à l'aide de moyens de numérisation 71, comprenant par exemple une caméra numérique couleur CCD avec résolution d'au moins 12,2 Mégapixels, un logiciel ImageJ V1, etc., de manière à constituer une image numérique de cet échantillon.

L'image numérique est alors chargée dans un processeur informatique 72 comprenant des moyens d'analyse numérique (non représentés) et des moyens de calcul de proportion (non représentés).

Les moyens d'analyse numérique sont agencés pour rechercher sur l'image numérique la présence éventuelle d'au moins deux types de métaux dans l'échantillon de fragments métalliques (étape d'analyse numérique).

La recherche sur l'image numérique de présence éventuelle d'au moins deux types de métaux dans l'échantillon de fragments métalliques comprend de préférence un ajustement de seuil de couleur et/ou de saturation et/ou de luminosité de l'image numérique, au moins lorsque le procédé est mis en œuvre la première fois pour un type d'échantillon donné ou pour une session d'analyse donnée.

Pour réaliser l'étape d'analyse numérique, on peut tout d'abord exclure l'arrière-fond de l'image, en l'occurrence le support 6. A cette fin, le support 6 peut être choisi d'une couleur pouvant être facilement distinguée de l'échantillon de fragments métalliques 88 étalé sur ce support 6. Ensuite, on peut ajuster des seuils d'analyse en sélectionnant sur l'image numérique les pixels qui présentent des couleurs typiques du premier type de, métal non ferreux lourd, par exemple, rouge et/ou jaune et/ou gris foncé. On peut assumer sur cette base que les pixels de couleur gris clair et/ou blanc correspondent à un métal non ferreux léger.

La sélection des pixels est faite en changeant les valeurs de couleur, de saturation et de luminosité sur la base d'une calibration. La calibration est faite avec un échantillon analysé en laboratoire.

Les moyens de calcul de proportion sont agencés pour calculer des quantités relatives entre lesdits au moins deux types de métaux et/ou pour calculer un rapport de quantités relatives entre lesdits au moins deux types de métaux (étape de calcul de proportion).

Lors de l'étape de calcul de proportion, on peut calculer le rapport $P_{i,j}$ de quantités relatives entre lesdits au moins deux types de métaux avec la formule suivante :

$$P_{i,j} = \frac{np_i * d_i}{np_i * d_i + np_j * d_j}$$

où : i est un premier type de métal, par exemple un métal non ferreux lourd, j est un deuxième type de métal, par exemple un métal non ferreux léger, $np_i$ est un nombre de pixels de l'image numérique correspondant au premier type de métal d'après l'étape d'analyse numérique, par exemple rouge et/ou jaune et/ou gris foncé, $np_j$ est un nombre de pixels de l'image numérique correspondant au deuxième type de métal d'après l'étape d'analyse numérique, par exemple gris clair et/ou blanc, $d_i$ correspond à la densité réelle du premier type de métal, $d_j$ correspond à la densité réelle du deuxième type de métal.

Tests réalisés avec différents acides

Trois solutions acides comprenant de l'acide nitrique ont été testées, ces solutions ayant une concentration respective de 1 %, 5 % et 10 %. Ce test a montré que la solution ayant une concentration de 5 % (et même entre 4 % et 6 % via d'autres tests) permettait de trouver un bon compromis entre une concentration limitée de la solution d'une part, et d'autre part le besoin d'obtenir une réaction forte avec les impuretés minérales contenues dans un échantillon immergé dans la solution.

L'acide nitrique a ensuite été testé comparativement avec quatre autres acides, chacun à une concentration de 5 %. Un tel test comparatif permet notamment de vérifier l'effet de ces différents acides sur l'étape d'analyse numérique du procédé, en particulier en termes de contraste de différents types de métaux présents dans un échantillon analysé, en particulier entre des métaux non ferreux lourds versus légers. Un objectif est d'augmenter un tel contraste afin d'identifier et/ou de quantifier les quantités relatives entre ces différents types de métaux. En effet, des métaux de différents types peuvent apparaître sur l'image numérique avec une couleur similaire, tel que typiquement l'aluminium et le zinc.

Le test comparatif a été mis en œuvre sur des échantillons de fragments métalliques comprenant des alliages d'aluminium et de zinc dans des proportions connues grâce à des analyses préalables réalisées à l'aide d'un système d'analyse XRF portable.

Les cinq solutions acides testées ont été préparées à partir de solutions standards concentrées. Ces cinq solutions acides sont l'acide nitrique $HNO_3$, l'acide chlorhydrique HCl, l'acide citrique $C_6H_8O_7$, l'acide phosphorique $H_3PO_4$ et l'acide sulfurique $H_2SO_4$.

Les échantillons de fragments métalliques ont été immergés chacun dans l'une des cinq solutions acides pendant cinq minutes, puis rincés avec de l'eau et sécher avec du papier.

Ce test comparatif a été réalisé trois fois.

Tous résultats confondus, ce test a montré :

- Que les alliages d'aluminium et les alliages de zinc apparaissent chacun avec une couleur grise sur une image numérique réalisée avant traitement acide, cela pour chacune des cinq solutions acides ;
- Que le traitement acide des échantillons tend à accentuer le contraste, sur l'image numérique réalisée après traitement acide, entre les deux types de métaux, et en particulier que ce traitement tend à rendre les alliages de zinc plus sombres sur l'image numérique par rapport aux alliages d'aluminium ; contrairement aux autres acides, l'acide nitrique a produit un tel résultat de manière consistante dans chacune des trois réalisations de ce test comparatif.

Ces différents tests montrent l'efficacité de l'acide nitrique à une concentration de 5 %, entre 4 % et 6 %, dans le procédé de l'invention.

Essai de mise en œuvre du procédé de l'invention

Compte-tenu des résultats ci-dessus, un essai de mise en œuvre du procédé de l'invention a été réalisé avec une solution acide comprenant de l'acide nitrique. Dans cet essai, un amas de fragments de métaux non ferreux de 0-20 mm ayant une masse d'environ 15 kg a été divisé en quatre échantillons. Trois de ces échantillons sont utilisés de la manière suivante.

*Premier échantillon (ou échantillon de référence) :*

Un premier échantillon, servant de référence, a été analysé en laboratoire selon un protocole d'analyse standard, tel que décrit par exemple dans le document Renvoise L. (2014), 19003 REM00 - PRECIOUS IBA - task 2.1 Analytical Campaign - Methodology Analysis (pp. 1-28). Notamment, cet échantillon a été soumis à des opérations de concassage et de tamisage afin d'en extraire au mieux les fragments non métalliques. Pour ce faire, deux concasseurs ont été utilisés générant respectivement des fragments ayant une taille de grain inférieure à 4 mm et inférieure à 1 mm. Les fragments métalliques ont ensuite été divisés en une fraction grossière (fragments > 10 mm) et une fraction fine (fragments < 10 mm). La fraction fine a été analysée en un seul bloc à l'aide d'un système d'analyse XRF, tandis que la fraction grossière a été analysée fragment par fragment à l'aide d'un système d'analyse XRF portable. De plus, en laboratoire, la densité réelle a été mesurée et les résultats ont été de 6,4 g/cm$^3$ pour les métaux non ferreux lourds et de 2,4 g/cm$^3$ pour les métaux non ferreux légers. Ces densités ont été utilisées, pour les analyses décrites ci-dessous, en tant que densité réelle (voir $d_i$ et $d_j$ dans la formule ci-dessous). En outre, cette analyse en laboratoire a permis d'établir que l'échantillon contenait, à l'issue des opérations de tamisage et de concassage, 79 % de métaux non ferreux légers et 21 % de métaux non ferreux lourds en excluant tous les fragments non métalliques. En incluant les fragments non métalliques, l'échantillon contenait 60 % de fragments non métalliques, 32 % de métaux non ferreux légers et 8 % de métaux non ferreux lourds.

*Deuxième échantillon (comparaison selon la concentration en HNO$_3$) :*

En référence à la figure, un deuxième échantillon 84 a été soumis à l'étape de purification :

- par tamisage à l'aide du tamis 32 ayant des ouvertures de 1 mm afin d'éliminer les très fines particules, puis
- par concassage à l'aide d'un concasseur à mâchoires 31.

Ces opérations de tamisage et de concassage ont été réalisées à trois reprises en modifiant progressivement la taille maximale de concassage de 10 à 5 mm. Le concassage a pour objectif de pulvériser les fragments non métalliques et de libérer ainsi les fragments métalliques. La taille maximale de concassage a été réduite à 5 mm pour simuler un concassage réduit comme celui qui peut être réalisé sur site.

A l'issue de ces opérations, la partie restante du deuxième échantillon a été divisée en trois sous-échantillons. Chacun de ces sous-échantillons a été traité, séparément, comme suit. Le sous-échantillon 85, placé sur un tamis (non représenté) ayant des ouvertures de 1 mm, a été rincé à l'eau à l'aide du premier moyen de rinçage 41, par projection d'eau sur la surface de ce tamis. Le sous-échantillon 86 ainsi rincé a ensuite été immergé dans une solution acide à l'aide des moyens de nettoyage par traitement acide 5. La concentration en acide nitrique était différente pour chacun des trois sous-échantillons : 1 % pour le premier, 5 % pour le deuxième et 10 % pour le troisième. Pour chacun des sous-échantillons, le ratio entre le volume de la solution acide et la masse du sous-échantillon 86 était d'environ 0,5 l/kg. Après rinçage à l'eau du sous-échantillon 87 ainsi traité à l'aide du deuxième moyen de rinçage 42, le sous-échantillon 88 ainsi rincé a été étalé sur le support 6 de manière à réduire les chevauchements entre ses différents fragments. La couleur du support 6 a été choisie de manière à pouvoir distinguer le support 6 sur l'image numérique, par rapport au sous-échantillon 88. Pour chacun des trois sous-échantillons, une image numérique a été réalisée à l'aide des moyens de numérisation 71.

Pour chaque image numérique associée respectivement à chacun des sous-échantillons, l'image numérique a été chargée dans le processeur informatique 72. L'arrière-fond de l'image a tout d'abord été isolé du sous-échantillon. Ensuite, en ajustant les seuils de couleur, de saturation et de luminosité, des fragments rouges représentant des fragments de métaux non ferreux lourds ont été recherchés sur l'image numérique. La proportion P de fragments de métaux non ferreux lourds dans le sous-échantillon a été calculée avec la formule suivante :

$$P = \frac{np_i * d_i}{np_i * d_i + np_j * d_j}$$

$np_i$ étant le nombre de pixels rouges sur l'image numérique, $np_j$ étant le nombre de pixels gris clair sur l'image numérique, $d_i$ correspondant à la densité réelle des métaux non ferreux lourds purs (6,4 g/cm$^3$), $d_j$ correspond à la densité réelle des métaux non ferreux légers purs (2,4 g/cm$^3$).

Des résultats de laboratoire ont été utilisés pour calibrer les seuils de couleur, de saturation et de luminosité pour l'analyse du deuxième sous-échantillon (concentration en acide nitrique de 5 %). Le même paramétrage a été utilisé pour l'analyse des deux autres sous-échantillons (concentration en acide nitrique de 1 % et 10 % respectivement).

Concernant ce deuxième échantillon, les fragments non métalliques représentaient seulement 16 % de sa composition à l'issue des opérations de tamisage et de concassage, indiquant que le concassage plus grossier, par comparaison avec l'échantillon de référence, entraîne une sous-estimation de la quantité des fragments non métalliques dans l'échantillon. Néanmoins, le calcul des quantités relatives entre les métaux non ferreux lourds et les métaux non ferreux légers contenus dans ce deuxième échantillon a permis d'obtenir un résultat comparable aux résultats obtenus en laboratoire avec l'échantillon de référence (voir tableau 1).

| Tableau 1 : proportion calculée de métaux non ferreux lourds et légers dans le deuxième échantillon | | | |
|---|---|---|---|
| | Premier sous-échantillon (concentration HNO$_3$ = 1 %) | Deuxième sous-échantillon (concentration HNO$_3$ = 5 %) | Troisième sous-échantillon (concentration HNO$_3$ = 10 %) |
| Métaux non ferreux lourds | 28 % | 21 % | 26 % |
| Métaux non ferreux légers | 72 % | 79 % | 74 % |

Les résultats de l'analyse de ce deuxième échantillon, résumés dans le tableau 1, sont très proches des résultats de l'analyse de l'échantillon de référence en laboratoire.

*Troisième échantillon (échantillon non concassé) :*

Un troisième échantillon a été utilisé pour tester le procédé de l'invention sur un échantillon non concassé. En référence à la figure, une fraction 85 de 0,4 kg de ce troisième échantillon a été rincée à l'eau à l'aide du premier moyen de rinçage 41 puis immergée pendant cinq minutes dans une solution acide (moyens de nettoyage par traitement acide 5) ayant une concentration de 5 % en acide nitrique, puis à nouveau rincée à l'aide du deuxième moyen de rinçage 42. La mise en œuvre des étapes de numérisation et d'analyse numérique a montré que l'étape de nettoyage par traitement acide d'une telle fraction d'échantillon non concassé est relativement peu efficace pour révéler les couleurs d'au moins deux types de métaux.
Il en résulte que le procédé de l'invention se montre davantage efficace lorsque l'échantillon de fragments métalliques analysé a été préalablement concassé, au moins lorsque la concentration en acide nitrique est de 5 %.

*Echantillons supplémentaires (comparaison selon la taille des fragments) :*

Le procédé a en outre été testé sur des échantillons supplémentaires de métaux non ferreux lourds qui ont été collectés et analysés dans le cadre d'autres études. Ces échantillons supplémentaires comprenaient respectivement des fragments métalliques ayant une taille de 6-10 mm (ES1), 10-15 mm (ES2) et 15-20 mm (ES3). Ces échantillons supplémentaires ne contenaient pas de fragments non métalliques. Chacun des échantillons supplémentaires a été immergé pendant cinq minutes dans une solution acide (moyens de nettoyage par traitement acide 5) ayant une concentration de 5 % en acide nitrique. Pour chacun des échantillons supplémentaires, le ratio entre le volume de la solution acide et la masse d'un échantillon supplémentaire donné était compris entre 0,4 et 0,5 l/kg. Les étapes de numérisation, d'analyse numérique et de calcul de proportion ont été mises en œuvre avec ces échantillons supplémentaires.
Le calcul des quantités relatives entre les métaux non ferreux lourds et les métaux non ferreux légers contenus dans chacun de ces échantillons supplémentaires a permis d'obtenir un résultat proche (écarts inférieurs à 5 %) des résultats obtenus par des tests de laboratoire traditionnels (voir tableau 2).

| Tableau 2 : proportion calculée de métaux non ferreux lourds dans les échantillons supplémentaires ES1, ES2 et ES3 et résultats de laboratoire | | | |
|---|---|---|---|
| | ES1 (6-10 mm) | ES2 (10-15 mm) | ES3 (15-20 mm) |
| Proportion de métaux non ferreux lourds | 98 % | 97 % | 57 % |
| Résultats de laboratoire | 99 % | 98 % | 55 % |

Le procédé selon l'invention permet d'obtenir un résultat d'analyse rapide. Par comparaison avec des analyses en laboratoire impliquant plusieurs jours de travail pour un technicien de laboratoire, le procédé de l'invention ne nécessite qu'une ou deux heures pour traiter l'échantillon et quelques minutes pour réaliser les étapes de numérisation, d'analyse numérique et de calcul de proportion.
Bien sûr, l'invention n'est pas limitée aux exemples qui viennent d'être décrits et de nombreux aménagements peuvent être apportés à ces exemples sans sortir du cadre de l'invention. Par exemple, l'échantillon à analyser peut subir différentes opérations préalables à l'étape de nettoyage par traitement acide, par exemple une ou plusieurs opérations de concassage et/ou de filtration et/ou de réduction de masse et/ou de rinçage... De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

**Revendications**

1. Procédé d'analyse de métaux comprenant une étape de nettoyage par traitement acide (5) dans laquelle un échantillon de fragments métalliques (86) est immergé dans une solution acide comprenant de l'acide nitrique et fournissant un échantillon de fragments métalliques (88) nettoyé, **caractérisé en ce qu'**il comprend en outre :

   - une étape de numérisation (71) dans laquelle ledit échantillon de fragments métalliques (88) nettoyé est numérisé de manière à constituer une image numérique de cet échantillon,
   - une étape d'analyse numérique (72) dans laquelle on recherche sur ladite image numérique la présence éventuelle d'au moins deux types de métaux dans l'échantillon de fragments métalliques nettoyé (88),
   - une étape de calcul de proportion (72) dans laquelle on calcule des quantités relatives entre lesdits au moins deux types de métaux ou un rapport de quantités relatives entre lesdits au moins deux types de métaux, lesdits au moins deux métaux comprenant un premier type de métal, ledit premier type de métal étant un métal non ferreux léger dont la densité est inférieure à 2800 kg/m$^3$, et un deuxième type de métal, ledit deuxième type de métal étant un métal non ferreux lourd dont la densité est supérieure à 2800 kg/m$^3$.

2. Procédé selon la revendication 1, **caractérisé en ce que** la recherche sur l'image numérique de présence éventuelle d'au moins deux types de métaux dans l'échantillon de fragments métalliques nettoyé (88) comprend un ajustement de seuil de couleur et/ou de saturation et/ou de luminosité de l'image numérique.

3. Procédé selon la revendication 1 ou 2, **caractérisé en ce que** le premier type de métal comprend de l'aluminium et **en ce que** le deuxième type de métal comprend du zinc et/ou du cuivre.

4. Procédé selon l'une quelconque des revendications 1 à 3, **caractérisé en ce que** l'échantillon de fragments métalliques (86) est issu de résidus d'incinération de déchets.

5. Procédé selon l'une quelconque des revendications 1 à 4, **caractérisé en ce qu'**il comprend en outre, avant l'étape de nettoyage par traitement acide (5), une étape de purification (31, 32) dans laquelle des impuretés sont extraites de l'échantillon de fragments métalliques (84).

6. Procédé selon la revendication 5, **caractérisé en ce que** les impuretés comprennent des fragments non métalliques.

7. Procédé selon l'une quelconque des revendications 1 à 6, **caractérisé en ce qu'**il comprend en outre, avant l'étape de numérisation (71) et après l'étape de nettoyage par traitement acide (5), une étape d'étalement dans laquelle l'échantillon de fragments métalliques (88) est étalé sur un support (6) de manière à réduire des chevauchements entre différents fragments de l'échantillon de fragments métalliques.

8. Procédé selon l'une quelconque des revendications 1 à 7, **caractérisé en ce que**, lors de l'étape de calcul de proportion (72), le rapport $P_{i,j}$ de quantités relatives entre lesdits au moins deux types de métaux est calculé avec la formule suivante :

$$P_{i,j} = \frac{np_i * d_i}{np_i * d_i + np_j * d_j}$$

où : $i$ est un premier type de métal, $j$ est un deuxième type de métal, $np_i$ est un nombre de pixels de l'image numérique correspondant au premier type de métal d'après l'étape d'analyse numérique, $np_j$ est un nombre de pixels de l'image numérique correspondant au deuxième type de métal d'après l'étape d'analyse numérique, $d_i$ correspond à la densité réelle du premier type de métal, $d_j$ correspond à la densité réelle du deuxième type de métal.

9. Installation d'analyse de métaux comprenant des moyens de nettoyage par traitement acide (5) agencés pour immerger un échantillon de fragments métalliques (86) dans une solution acide comprenant de l'acide nitrique et pour fournir un échantillon de fragments métalliques (88) nettoyé, **caractérisé en ce qu'**elle comprend en outre :

- des moyens de numérisation (71) agencés pour numériser l'échantillon de fragments métalliques nettoyé (88) de manière à constituer une image numérique de cet échantillon,
- des moyens d'analyse numérique (72) agencés pour rechercher sur l'image numérique la présence éventuelle d'au moins deux types de métaux dans l'échantillon de fragments métalliques nettoyé (88),
- des moyens de calcul de proportion (72) agencés pour calculer des quantités relatives entre lesdits au moins deux types de métaux ou un rapport de quantités relatives entre lesdits au moins deux types de métaux, lesdits au moins deux de métaux comprenant un premier type de métal, ledit premier type de métal étant un métal non ferreux léger dont la densité est inférieure à 2800 kg/m$^3$, et un deuxième type de métal, ledit deuxième type de métal étant un métal non ferreux lourd dont la densité est supérieure à 2800 kg/m$^3$.

**Patentansprüche**

1. Verfahren zur Analyse von Metallen, umfassend einen Schritt der Reinigung durch Säurebehandlung (5), bei dem eine Probe von Metallfragmenten (86) in eine Säurelösung, die Salpetersäure umfasst, getaucht wird und eine gereinigte Probe von Metallfragmenten (88) erhalten wird, **dadurch gekennzeichnet, dass** es weiterhin umfasst:

- einen Schritt der Digitalisierung (71), bei dem die gereinigte Probe von Metallfragmenten (88) digitalisiert wird, so dass ein digitales Bild dieser Probe entsteht;
- einen Schritt der digitalen Analyse (72), bei dem man auf dem digitalen Bild die mögliche Anwesenheit von wenigstens zwei Arten von Metallen in der gereinigten Probe von Metallfragmenten (88) sucht;
- einen Schritt der Verhältnisrechnung (72), bei dem man relative Mengen zwischen den wenigstens zwei Arten von Metallen oder ein Verhältnis von relativen Mengen zwischen den wenigstens zwei Arten von Metallen berechnet, wobei die wenigstens zwei Metalle eine erste Art Metall, wobei die erste Art Metall ein leichtes Nichteisenmetall ist, dessen Dichte kleiner als 2800 kg/m$^3$ ist, und eine zweite Art Metall, wobei die zweite Art Metall ein schweres Nichteisenmetall ist, dessen Dichte größer als 2800 kg/m$^3$ ist, umfassen.

2. Verfahren gemäß Anspruch 1, **dadurch gekennzeichnet, dass** die Suche der möglichen Anwesenheit von wenigstens zwei Arten von Metallen in der gereinigten Probe von Metallfragmenten (88) auf dem digitalen Bild eine Justierung der Farb- und/oder Sättigungs- und/oder Helligkeitsschwelle des digitalen Bildes umfasst.

3. Verfahren gemäß Anspruch 1 oder 2, **dadurch gekennzeichnet, dass** die erste Art Metall Aluminium umfasst, und dadurch, dass die zweite Art Metall Zink und/oder Kupfer umfasst.

4. Verfahren gemäß einem der Ansprüche 1 bis 3, **dadurch gekennzeichnet, dass** die Probe von Metallfragmenten (86) aus Rückständen der Müllverbrennung stammt.

5. Verfahren gemäß einem der Ansprüche 1 bis 4, **dadurch gekennzeichnet, dass** es weiterhin vor dem Schritt der Reinigung durch Säurebehandlung (5) einen Schritt der Reinigung (31, 32) umfasst, bei dem Verunreinigungen aus der Probe von Metallfragmenten (84) extrahiert werden.

6. Verfahren gemäß Anspruch 5, **dadurch gekennzeichnet, dass** die Verunreinigungen nichtmetallische Fragmente umfassen.

7. Verfahren gemäß einem der Ansprüche 1 bis 6, **dadurch gekennzeichnet, dass** es weiterhin vor dem Schritt der Digitalisierung (71) und nach dem Schritt der Reinigung durch Säurebehandlung (5) einen Schritt der Ausbreitung umfasst, bei dem die Probe von Metallfragmenten (88) auf einem Träger (6) ausgebreitet wird, so dass Überlappungen zwischen verschiedenen Fragmenten der Probe von Metallfragmenten reduziert werden.

8. Verfahren gemäß einem der Ansprüche 1 bis 7, **dadurch gekennzeichnet, dass** in dem Schritt der Verhältnisrechnung (72) das Verhältnis $P_{i,j}$ von relativen Mengen zwischen den wenigstens zwei Arten von Metallen mit der folgenden Formel berechnet wird:

**EP 3 163 163 B1**

$$P_{i,j} = \frac{np_i * d_i}{np_i * d_i + np_j * d_j},$$

wobei i eine erste Art Metall ist, j eine zweite Art Metall ist, $np_i$ die Anzahl der Pixel des digitalen Bildes, die der ersten Art Metall entsprechen, gemäß dem Schritt der digitalen Analyse ist, $np_j$ die Anzahl der Pixel des digitalen Bildes, die der zweiten Art Metall entsprechen, gemäß dem Schritt der digitalen Analyse ist, $d_i$ der wahren Dichte der ersten Art Metall entspricht, $d_j$ der wahren Dichte der zweiten Art Metall entspricht.

9.  Anlage zur Analyse von Metallen, umfassend Einrichtungen zur Reinigung durch Säurebehandlung (5), die konfiguriert sind, um eine Probe von Metallfragmenten (86) in eine Säurelösung, die Salpetersäure umfasst, zu tauchen und eine gereinigte Probe von Metallfragmenten (88) zu erhalten, **dadurch gekennzeichnet, dass** sie weiterhin umfasst:

    - Einrichtungen zur Digitalisierung (71), die konfiguriert sind, um die gereinigte Probe von Metallfragmenten (88) zu digitalisieren, so dass ein digitales Bild dieser Probe entsteht;
    - Einrichtungen zur digitalen Analyse (72), die konfiguriert sind, um auf dem digitalen Bild die mögliche Anwesenheit von wenigstens zwei Arten von Metallen in der gereinigten Probe von Metallfragmenten (88) zu suchen;
    - Einrichtungen zur Verhältnisrechnung (72), die konfiguriert sind, um relative Mengen zwischen den wenigstens zwei Arten von Metallen oder ein Verhältnis von relativen Mengen zwischen den wenigstens zwei Arten von Metallen zu berechnen, wobei die wenigstens zwei Metalle eine erste Art Metall, wobei die erste Art Metall ein leichtes Nichteisenmetall ist, dessen Dichte kleiner als 2800 kg/m$^3$ ist, und eine zweite Art Metall, wobei die zweite Art Metall ein schweres Nichteisenmetall ist, dessen Dichte größer als 2800 kg/m$^3$ ist, umfassen.

**Claims**

1.  Process for analyzing metals comprising an acid treatment cleaning step (5) in which a sample of metal fragments (86) is immersed in an acid solution, comprising nitric acid and leading to a cleaned sample of metal fragments (88) **characterized in that** it additionally comprises:

    - a digitizing step (71) in which the cleaned sample of metal fragments (88) is digitized so as to form a digital image of this sample;
    - a numerical analysis step (72) in which the optional presence of at least two types of metals in the cleaned sample of metal fragments (88) is searched for on the digital image;
    - a proportion calculation step (72) in which relative amounts between said at least two types of metals or a ratio of relative amounts between said at least two types of metals are calculated, said at least two metals comprising a first type of a metal, said first type of metal being a light non-ferrous metal of which the density is less than 2800 kg/m$^3$, and a second type of metal that is a heavy non-ferrous metal, said second type of metal being a heavy non-ferrous metal of which the density is greater than 2800 kg/m$^3$.

2.  Process according to Claim 1, **characterized in that** the search, on the digital image, for the optional presence of at least two types of metals in the cleaned sample of metal fragments(88) comprises an adjustment of colour threshold and/or saturation threshold and/or luminosity threshold of the digital image.

3.  Process according to Claim 1 or 2, **characterized in that** the first type of metal comprises aluminium and the second type of metal comprises zinc and/or copper

4.  Process according to any one of Claims 1 to 3, **characterized in that** the sample of metal fragments (86) is derived from waste incineration residues.

5.  Process according to any one of Claims 1 to 4, **characterized in that** it also comprises, before the acid treatment cleaning step (5), a purification step (31, 32) in which impurities are extracted from the sample of metal fragments (84).

6.  Process according to Claim 5, **characterized in that** the impurities comprise non-metal fragments.

7.  Process according to any one of Claims 1 to 6, **characterized in that** it also comprises, before the digitizing step (71) and after the acid treatment cleaning step (5), a spreading step in which the sample of metal fragments (88) is spread over a support (6) so as to reduce overlaps between various fragments of the sample of metal fragments.

8. Process according to any one of Claims 1 to 7, **characterized in that**, during the proportion calculation step (72), the ratio $P_{i,j}$ of relative amounts between said at least two types of metals is calculated with the following formula:

$$P_{i,j} = \frac{np_i * d_i}{np_i * d_i + np_j * d_j}$$

where: $i$ is a first type of metal, $j$ is a second type of metal, $np_i$ is a number of pixels of the digital image corresponding to the first type of metal according to the numerical analysis step, $np_j$ is a number of pixels of the digital image corresponding to the second type of metal according to the numerical analysis step, $d_i$ corresponds to the real density of the first type of metal and $d_j$ corresponds to the real density of the second type of metal.

9. Plant for analysing metals comprising acid treatment cleaning means (5) arranged in order to immerse a sample of metal fragments (86) in an acid solution, comprising nitric acid and to give a cleaned sample of metal fragments (88) **characterized in that** it also comprises:

- digitizing means (71) arranged in order to digitize the cleaned sample of metal fragments (88) so as to form a digital image of this sample;
- numerical analysis means (72) arranged in order to search for, on the digital image, the optional presence of at least two types of metals in the cleaned sample of metal fragments (88);
- proportion calculation means (72) arranged in order to calculate relative amounts between said at least two types of metals or a ratio of relative amounts between said at least two types of metals, said at least two metals comprising a first type of a metal, said first type of metal being a light non-ferrous metal of which the density is less than 2800 kg/m$^3$, and a second type of metal that is a heavy non-ferrous metal, said second type of metal being a heavy non-ferrous metal of which the density is greater than 2800 kg/m$^3$.

## Figure unique

**EP 3 163 163 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SABIR SYED.** Optical and Chemical Methods of Metal Ash Analysis and Tin Recovery. *Environ Monit Assess,* 2007, vol. 130, 311-322
- **BONIFAZI ; SERRANTI.** Hyperspectral imaging based procédures applied to bottom ash characterization. *Advanced Environmental, Chemical and Biological Sensing Technologies V, Proc. of SPIE,* 2007, vol. 6755, 67550B
- **RENVOISE L.** 19003 REM00 - PRECIOUS IBA - task 2.1 Analytical Campaign. *Methodology Analysis,* 2014, 1-28